(19) Europäisches Patentamt
European Patent Office
Office européen des brevets

(11) Veröffentlichungsnummer: **0 217 024**
**B1**

(12) # EUROPÄISCHE PATENTSCHRIFT

(45) Veröffentlichungstag der Patentschrift: **25.04.90**

(51) Int. Cl.⁵: **C 07 D 291/06**

(21) Anmeldenummer: **86109944.8**

(22) Anmeldetag: **19.07.86**

(54) **Verfahren zur Herstellung von 6-Methyl-3,4-dihydro-1,2,3-oxathiazin-4-on-2,2-dioxid.**

(30) Priorität: **29.07.85 DE 3527070**

(43) Veröffentlichungstag der Anmeldung:
**08.04.87 Patentblatt 87/15**

(45) Bekanntmachung des Hinweises auf die Patenterteilung:
**25.04.90 Patentblatt 90/17**

(84) Benannte Vertragsstaaten:
**AT BE CH DE FR GB IT LI NL SE**

(56) Entgegenhaltungen:
**EP-A-0 159 516**
**DE-A-2 434 549**
**DE-A-3 410 439**
**DE-B-2 327 804**

**ANGEWANDTE CHEMIE, Band 85, Nr. 22, 1973, Seiten 965-973, K. CLAUSS et al.: "Oxathiazinondioxide, eine neue Gruppe von Süssstoffen"**

(73) Patentinhaber: **HOECHST AKTIENGESELLSCHAFT**
**Postfach 80 03 20**
**D-6230 Frankfurt am Main 80 (DE)**

(72) Erfinder: **Schütz, Joachim, Dr.**
**Kurhausstrasse 57A**
**D-6238 Hofheim am Taunus (DE)**
Erfinder: **Schweikert, Otto Ernst, Dr.**
**Freiherr-vom-Stein-Strasse 37**
**D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 217 024 B1

Courier Press, Leamington Spa, England.

## Beschreibung

6-Methyl-3,4-dihydro-1,2,3-oxathiazin-4-on-2,2-dioxid ist die Verbindung der Formel

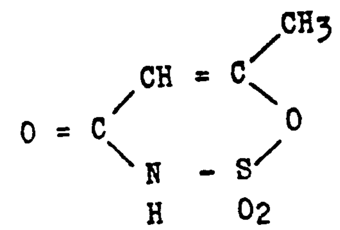

Infolge des aciden Wasserstoffs am Stickstoffatom ist die Verbindung zur Salzbildung (mit Basen) befähigt. Die nicht-toxischen Salze — wie z.B. das Na-, das K- und das Ca-Salz — können wegen ihres z.T. intensiven Süßgeschmacks als Süßstoffe auf dem Nahrungsmittelsektor verwendet werden, wobei das K-Salz ("Acesulfam K" oder auch nur "Acesulfam") von besonderer Bedeutung ist.

Zur Herstellung des 6-Methyl-3,4-dihydro-1,2,3-oxathiazin-4-on-2,2-dioxids und dessen nicht-toxischer Salze ist eine Reihe verschiedener Verfahren bekannt; vgl. Angewandte Chemie *85,* Heft 22 (1973) S. 965 bis 73, entsprechend International Edition Vol. 12, No. 11 (1973), S. 869—76. Praktisch alle Verfahren gehen von Chlor- oder Fluorsulfonyliso-cyanat ($XSO_2NCO$ mit X = Cl oder F) aus. Das Chlor- bzw. Fluorsulfonylisocyanat wird dann mit Monomethylacetylen, Aceton, Acetessigsäure, Acetessigsäuretert.-butylester oder Benzylpropenylether (in einer meist mehrstufigen Reaktion) zu Acetoacetamid-N-sulfochlorid bzw. -fluorid umgesetzt, was unter der Einwirkung von Basen (wie z.B. methanolischer KOH) cyclisiert und die entsprechenden Salze des 6-Methyl-3,4-dihydro-1,2,3-oxa-thiazin-4-on-2,2-dioxids liefert. Aus den Salzen kann das freie Oxathiazinon gewünschtenfalls auf übliche Weise (mit Säuren) erhalten werden.

Ein weiteres Verfahren zur Herstellung der Oxathiazinon-Zwischenstufe Acetoacetamid-N-sulfofluorid geht aus von Amidosulfofluorid $H_2NSO_2F$, dem partiellen Hydrolyseprodukt des Fluorsufonylisocyanats (DE—OS 2 453 063). Danach wird das Fluorid der Amidosulfonsäure $H_2NSO_2F$ mit einer etwa äquimolaren Menge des Acetoacetylierungsmittels Diketen in einem inerten organischen Lösungsmittel in Gegenwart eines Amins bei Temperaturen zwischen etwa −30 und 100°C umgesetzt; die Umsetzung verläuft nach folgender Reaktionsgleichung (mit Triethylamin als Amin):

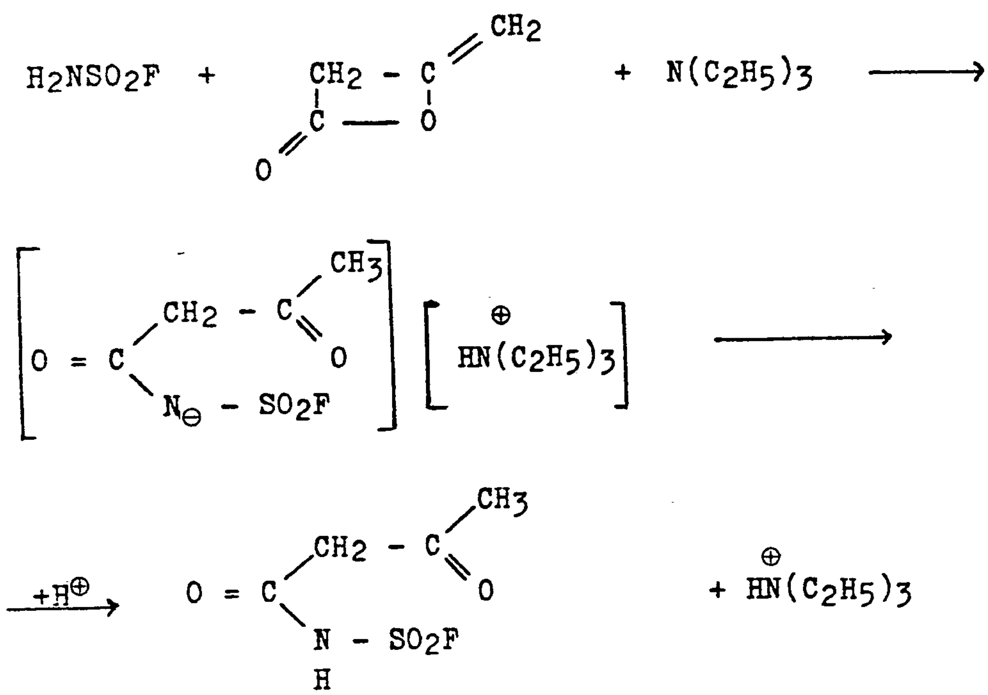

Acetoacetamid-N-sulfofluorid

Das Acetoacetamid-N-sulfofluorid wird dann auf übliche Weise mittels einer Base, z.B. mit methanolischer KOH, zum Süßstoff cyclisiert:

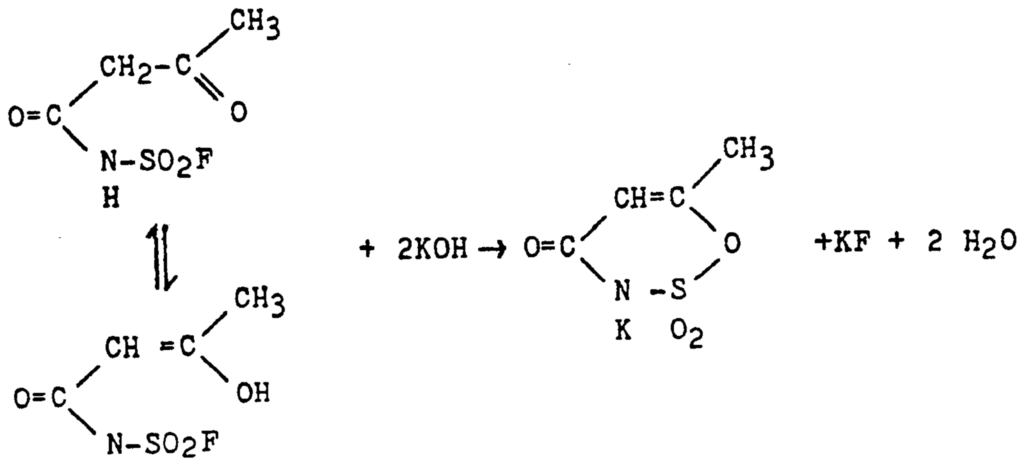

"Acesulfam"

Obwohl die bekannten Verfahren z.T. recht befriedigende Ausbeuten an 6-Methyl-3,4-dihydro-1,2,3-oxathiazin-4-on-2,2-dioxid und dessen nicht-toxischen Salzen liefern, (bis zu ca. 85% d.Th., bezogen auf das Ausgangs-Amidosulfonsäurehalogenid), sind sie wegen der Notwendigkeit des Einsatzes der nicht ganz einfach zugänglichen Ausgangsstoffe Chlor- bzw. Fluorsulfonylisocyanat vor allem für technische Belange noch verbesserungsbedürftig; die Herstellung des Chlor- und Fluor-sulfonylisocyanats erfordert nämlich wegen der z.T. ziemlich unangenehm handzuhabenden Ausgangsmaterialien (HCN, $Cl_2$, $SO_3$ und HF) erhebliche Vorsichtsmaßnahmen und Sicherheitsvorkehrungen. Der Herstellung des Chlor- und Fluor-sulfonylisocyanats liegen folgende Reaktionsgleichungen zugrunde:

$$HCN + Cl_2 \rightarrow ClCN + HCl$$
$$ClCN + SO_3 \rightarrow ClSO_2NCO$$
$$ClSO_2NCO + HF \rightarrow FSO_2NCO + HCl$$

Der Ersatz des Amidosulfofluorids in dem Verfahren gemäß der vorerwähnten DE—OS 24 53 063 etwa durch die wesentlich leichter (z.B. aus $NH_3 + SO_3$) erhältliche Amidosulfonsäure $H_2NSO_3H$ bzw. deren Salze erschien kaum erfolgversprechend, weil nämlich die Umsetzung des Na-Amidosulfonats $H_2NSO_3Na$ mit Diketen in wässrig-alkalischer Lösung überhaupt kein rein isolierbares Umsetzungsprodukt ergibt. Das bei dieser Umsetzung wohl zumindest mit entstandene 1:1-Addukt konnte veilmehr nur in Form des Kupplungsproduktes mit 4-Nitrophenyldiazoniumchlorid als blaßgelber Farbstoff gewonnen werden; vgl. Ber. *83* (1950), S. 551—558, insbesondere S. 555, letzter Absatz vor der Beschreibung der Versuche und S. 558, letzter Absatz:

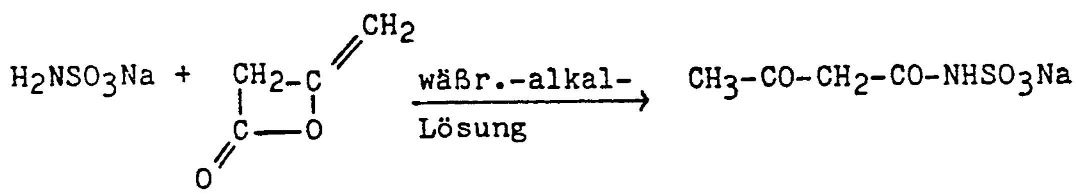


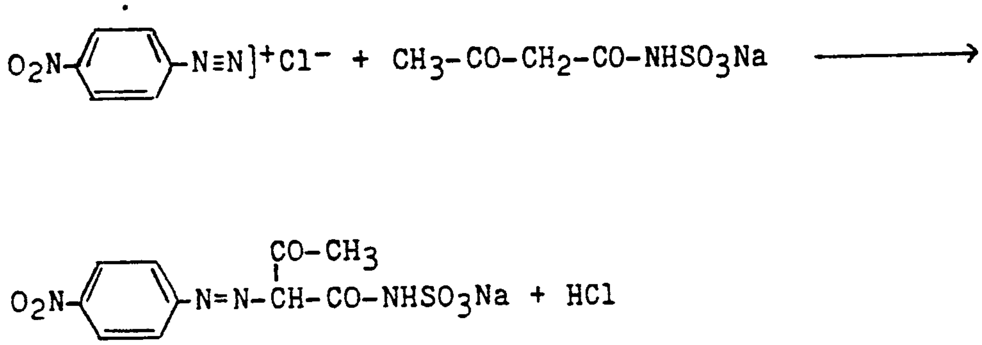

Die Acetoacetamid-N-sulfonsäure ist im übrigen ansonsten nur bzw. auch als Zwischenprodukt bei der Zersetzung des 6-Methyl-3,4-dihydro-1,2,3-oxathiazin-4-on-2,2-dioxids während des Kochens in wässriger Lösung postuliert worden; vgl. die anfangs zitierte Literatur Angew. Chemie (1973) a.a.O.:

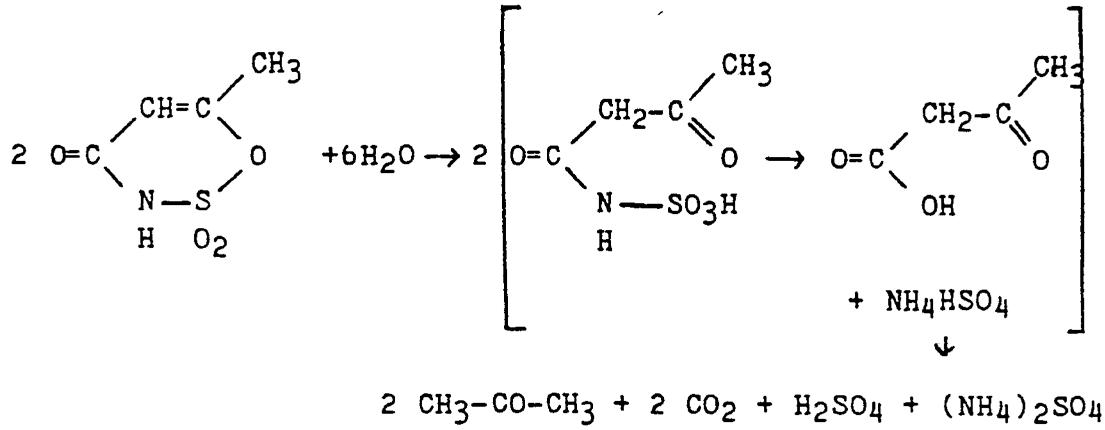

Wegen der insbesondere infolge der Notwendigkeit des Einsatzes nicht ganz einfach zugänglicher Ausgangsstoffe vor allem für die Durchführung in technischem Maßstab nicht ganz befriedigenden Verfahren des Standes der Technik zur Herstellung von 6-Methyl-3,4-dihydro-1,2,3-oxathiazin-4-on-2,2-dioxid und dessen nicht-toxischer Salze bestand somit die Aufgabe, die bekannten Verfahren entsprechend zu verbessern oder ein neues verbessertes Verfahren zu entwickeln.

Zur Lösung dieser Aufgabe wurde bereits vorgeschlagen, das Verfahren gemäß DE—OS 2 453 063 hauptsächlich in der Weise zu modifizieren, daß man das Amidosulfofluorid in dem bekannten Verfahren durch Salze der Amidosulfonsäure ersetzt und das erhaltene Acetoacetylierungsprodukt nachfolgend mittels $SO_3$ zum Ring schleißt (DE—A—3410439 oder EP—A—155634 mit Priorität 22.03.1984).

Die zuletzt genannte Patentanmeldung bezieht sich speziell auf ein Verfahren zur Herstellung von 6-Methyl-3,4-dihydro-1,2,3-oxathiazin-4-on-2,2-dioxid und dessen nichttoxischen Salzen durch

a) Umsetzung eines Amidosulfonsäurederivates mit einer mindestens etwa äquimolaren Menge eines Acetoacetylierungsmittels in einem inerten organischen Lösungsmittel, ggf. in Gegenwart eines Amin- oder Phosphin-Katalysators, zu einem Acetoacetamidderivat und

b) Ringschluß des Acetoacetamidderivats;

das Verfahren ist dadurch gekennzeichnet, daß man in Stufe a) als Amidosulfonsäurederivat ein in dem eingesetzten inerten organischen Lösungsmittel zumindest teilweise lösliches Salz der Amidosulfonsäure verwendet, daß man das in dieser Stufe gebildete Acetaoacetamid-N-sulfonat oder auch die freie Acetoacetamid-N-sulfonsäure in Stufe b) durch die Einwirkung der mindestens etwa äquimolaren Menge von $SO_3$, gegebenenfalls in einem inerten anorganischen oder organischen Lösungsmittel, zum Ring des 6-Methyl-3,4-dihydro-1,2-oxathiazin-4-on-2,2-dioxids schleißt und daß das hier in der Säureform anfallende Produkt dann gegebenenfalls noch in einer Stufe c) mit einer Base neutralisiert.

Als dem Verfahren zugrundeliegenden Reaktionsgleichungen sind in der vorerwähnten Patentanmeldung angegeben (mit Diketen als Acetoacetylierungsmittel):

a)

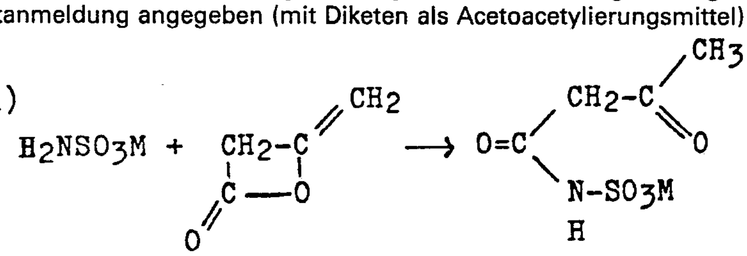

(M =Basenkation)

b)

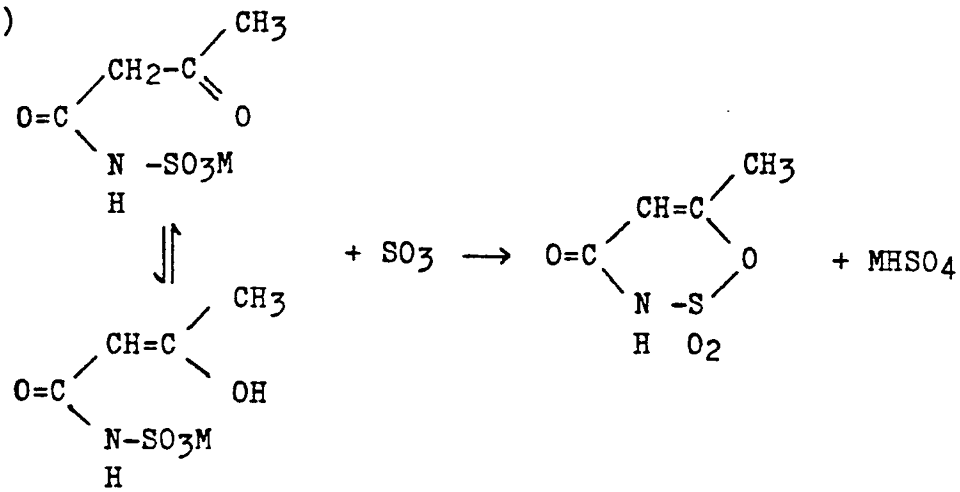

c)

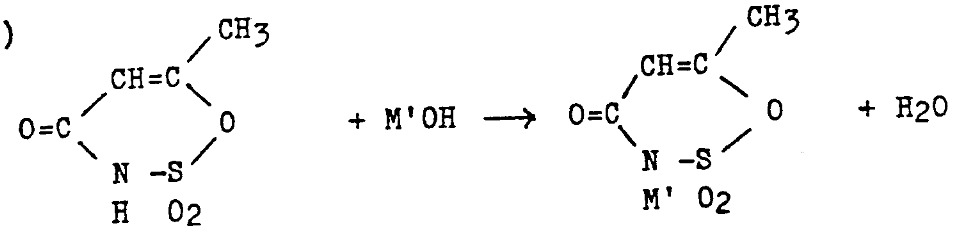

(M' = Basenkation)

In diesem Reaktionsschema ist Stufe b) mit einer gegenüber dem Acetoacetamid-N-Sulfonat äquimolaren $SO_3$-Menge dargestellt. Bevorzugt wird jedoch ein $SO_3$-Überschuß verwendet. Dabei entsteht ein in seiner chemischen Struktur noch nicht genau bekanntes Zwischenprodukt, das jedoch möglicherweise ein $SO_3$-Addukt des 6-Methyl-3,4-dihydro-1,2,3-oxathiazin-4-on-2,2-dioxids — nachfolgend als "$SO_3$-Addukt" bezeichnet — darstellt, welches dann noch hydrolysiert werden muß. In diesem Falle besteht die vorerwähnte Reaktionsstufe b) also aus 2 Teilstufen, nämlich

<u>b1</u>: Ringschluß

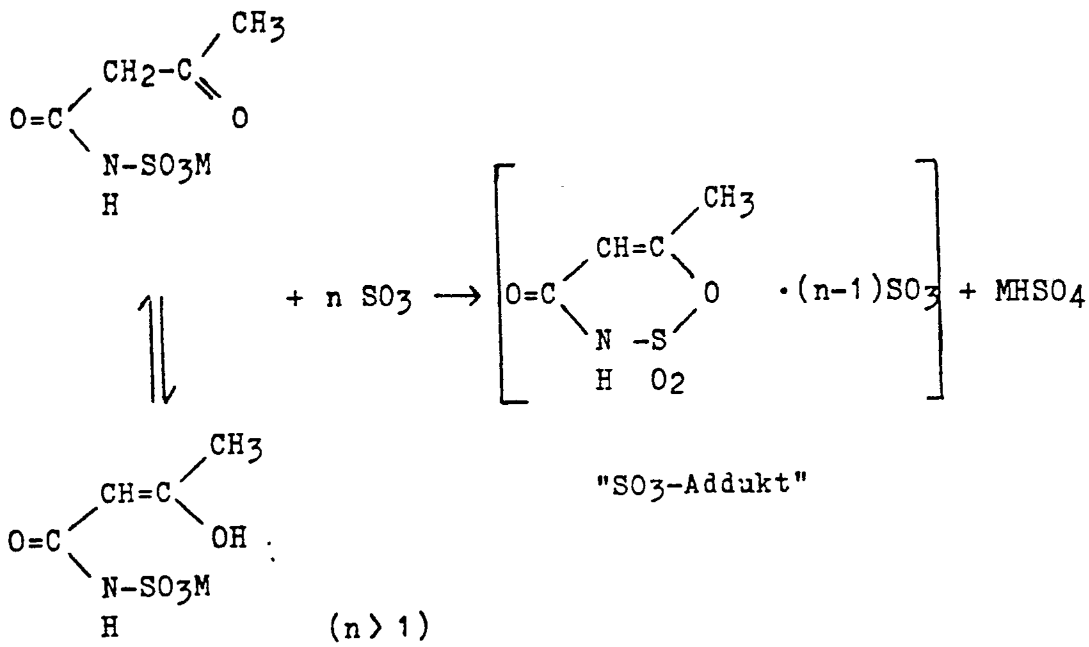


<u>b2</u>: Hydrolyse

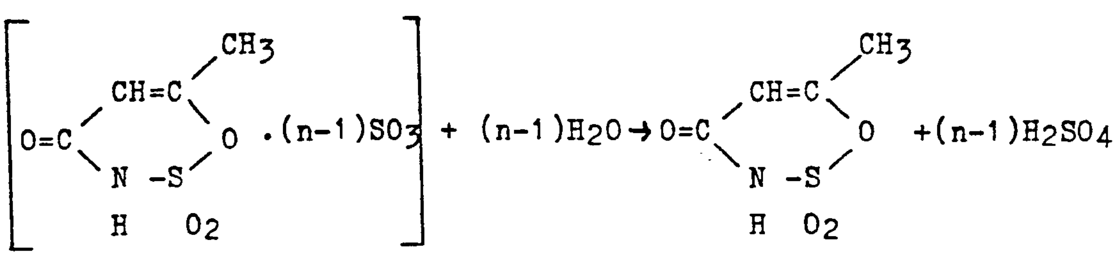

Als Reaktionstemperaturen für die Ringschlußreaktion (b1) sind in der vorerwähnten Patentanmeldung Temperaturen zwischen etwa −70 und +175°C, vorzugsweise zwischen etwa −40 und +10°C, angegeben. Die sich auf Reaktionsstufe b) beziehenden Beispiele arbeiten meist bei Temperaturen zwischen etwa −30 und −25°C.

Die Reaktionszeiten sollen "bis zu etwa 10 Stunden" betragen; eine Untergrenze ist nicht angegeben. In sämtlichen sich auf die Reaktionsstufe b) beziehenden Beispielen liegen die Reaktionszeiten der Ringschlußreaktion über einer Stunde; die Beispiele wurden durchweg in normalen Gefäßen unter Ruhren durchgeführt.

Die Hydrolyse (b2) erfolgt nach der Ringschlußreaktion durch Zugabe von Wasser oder Eis. Spezielle

Angaben bezüglich der Temperatur und der Reaktionszeit für die Hydrolyse sind in der vorerwähnten Patentanmeldung nicht enthalten.

Hinsichtlich der weiteren Verfahrensdetails wird auf die ausführliche Beschreibung in der genannten Patentanmeldung verwiesen.

Das Verfahren geht von einfach zugänglichen und wohlfeilen Ausgangsstoffen aus und ist außerordentlich einfach durchführbar. Die Ausbeuten liegen

in Stufe a) bei etwa 90 bis 100% d.Th. (bezogen auf das Ausgangs-Amidosulfonat),
in Stufe b) bei etwa 70 bis 95% d.Th. (bezogen auf das Acetoacetamid-N-sulfonat) und
in Stufe c) bei etwa 100% d.Th. (bezogen auf das Oxathazinon in der Säureform),
so daß für das Gesamtverfahren Ausbeuten zwischen etwa 65 und 95% d.Th. resultieren.

Im Zuge der weiteren Bearbeitung dieses Verfahrens wurde nun gefunden, daß sowohl die Ringschlußreaktion (b1) als auch die Hydrolyse (b2) in außerordentlich kurzen Zeiten ablaufen; dadurch läßt sich die technische Durchführung und insbesondere die Raum-Zeit-Ausbeute des Verfahrens erheblich verbessern.

Erfindungsgegenstand ist daher ein Verfahren zur Herstellung von 6-Methyl-3,4-dihydro-1,2,3-oxathiazin-4-on-2,2-dioxid durch Ringschluß eines Acetoacetamid-Derivats; das Verfahren ist dadurch gekennzeichnet, daß man als Acetoacetamid-Derivat Acetoacetamid-N-sulfonsäure oder deren Salze — gelöst in einem inerten Lösungsmittel — verwendet,

daß man den Ringschluß durch die Einwirkung einer mehr als äquimolaren Menge $SO_3$ — gegebenenfalls ebenfalls gelöst in einem interten Lösungsmittel — in einen Reaktorverweilzeit von höchstens 10 Minuten durchführt und daß man das als $SO_3$-Addukt anfallende 6-Methyl-3,4-dihydro-1,2,3-oxathiazin-4-on-2,2-dioxid dann hydrolysiert.

Das glatte Gelingen des Ringschlusses der Acetoacetamid-N-sulfonsäure und von deren Salzen mit $SO_3$ ist sehr überaschend, weil die unter Ringschluß erfolgende Wasser-bzw. Basen-Abspaltung nämlich mit anderen Wasser- bzw Basen-abspaltenden Mitteln wie z.B. $P_2O_5$, Acetanhydrid, Trifluoressigsäureanhydrid, Thionylchlorid etc. nicht oder jedenfalls praktisch nicht gelingt, wie bereits in der vorerwähnten Patentanmeldung anhand eines Vergleichsbeispiels (mit $P_2O_5$) gezeigt werden konnte.

Weiterhin ist auch der Reaktionsablauf in kurzen bis sehr kurzen Zeiten durchaus überraschend.

Wegen der kurzen Reaktionszeit und der hohen Wärmetönung werden hohe Ausbeuten bevorzugt in speziellen Vorrichtungen erzielt. Bevorzugte Verweilzeiten liegen zwischen etwa 0,001 und 60 Sekunden, insbesondere zwischen etwa 0,01 und 10 Sekunden.

Die Herstellung der Ausgangs-Acetamido-N-sulfonsäure und von deren Salzen erfolgt bevorzugt nach Stufe a) des Verfahrens der vorerwähnten Patentanmeldung durch Umsetzung der Li- oder Ammonium-Salze der Amidosulfonsäure mit Diketen in inerten organischen Lösungsmitteln. Hierbei werden Lösungen der Li- und Ammonium-Salze der Acetamido-N-sulfonsäure erhalten, welche als solche direkt für die Ringschlußreaktion mit $SO_3$ eingesetzt werden können.

Für die genannte Ringschlußreaktion können natürlich auch andere Salze der Acetamido-N-sulfonsäure — insbesondere Alkali — und Erdalkalisalze — verwendet werden. Der Einsatz der freien Acetamido-N-sulfonsäure bringt gegenüber den Salzen keine Vorteile.

Als inerte Lösungsmittel für die Lösung der Acetamido-N-sulfonsäure oder von deren Salzen kommen hauptsächlich diejenigen inerten organischen Lösungsmittel in Frage, welche in der vorerwähnten Patentanmeldung als Lösungsmittel für die Durchführung der Stufe a) des dortigen Verfahrens genannt sind; d.s. also

Halogenierte aliphatische Kohlenwasserstoffe, vorzugsweise solche mit bis zu 4 C-Atomen wie z.B. Methylenchlorid, Chloroform, 1,2-Dichlorethan, Trichlorethylen, Tetrachlorethylen, Trichlor-fluor-ethylen, etc.;

aliphatische Ketone, vorzugsweise solche mit 3 bis 6 C-Atomen wie z.B. Aceton, Methylethylketon etc.;

Aliphatische Ether, vorzugsweise cyclische aliphatische Ether mit 4 bis 5 C-Atomen wie z.B. Tetrahydrofuran, Dioxan etc.;

niedere aliphatische Carbonsäuren, vorzugsweise solche mit 2 bis 6 C-Atomen wie z.B. Essigsäure, Propionsäure etc.;

aliphatische Nitrile, vorzugsweise Acetonitril;

N-alkylsubstituierte Amide der Kohlensäure und niederer aliphatischer Carbonsäuren, vorzugsweise Amide mit bis zu 5 C-Atomen wie z.B. Tetramethylharnstoff, Dimethylformamid, Dimethylacetamid, N-Methylpyrrolidon, etc.;

aliphatische Sulfoxide, vorzugsweise Dimethylsulfoxid, und
aliphatische Sulfone, vorzugsweise Sulfolan

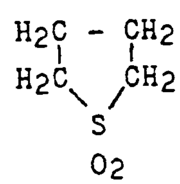

Besonders bevorzugte Lösungsmittel aus der vorstehenden Aufzählung sind Methylenchlorid, 1,2-Dichlorethan, Aceton, Eisessig und Dimethylformamid, vor allem Methylenchlorid.

Die Lösungsmittel können sowohl einzeln als auch in Mischung eingesetzt werden.

Die Konzentration der Acetoacetamid-N-sulfonsäure oder von deren Salzen im inerten Lösemittel ist nicht kritisch, wird aber einerseits begrenzt durch die Löslichkeit, andererseits durch Wirtschaftlichkeitsüberlegungen, da bei hoher Verdünnung viel Lösemittel nachher wieder abgetrennt und aufgearbeitet werden muß. Im allgemeinen sind Konzentrationen zwischen etwa 0,1 und 2 Mol Acetoacetamid-N-sulfonsäure oder von deren Salzen pro Liter zweckmäßig.

$SO_3$ kann gasförmig oder in flüssiger bzw. gelöster Form eingesetzt werden. Als Lösungsmittel für das $SO_3$ kommen die bei der Beschreibung der Stufe b) des Verfahrens der vorerwähnten Patentanmeldung genannten anorganischen und organischen Lösungsmittel in Frage; d.s. also

| | |
|---|---|
| Anorganische Lösungsmittel: | flüssiges $SO_2$; |
| organische Lösungsmittel: | halogenierte aliphatische Kohlenwasserstoffe, vorzugsweise mit bis zu 4 C-Atomen wie z.B. Methylenchlorid, Chloroform, 1,2-Dichlorethan, Trichloroethylen, Tetrachlorethylen, Trichlor-fluorethylen etc.; |

Kohlensäureester mit niederen aliphatischen Alkoholen, vorzugsweise mit Methanol oder Ethanol;
Nitroalkane, vorzugsweise mit bis zu 4 C-Atomen, insbesondere Nitromethan;
alkylsubstituierte Pyridine, vorzugsweise Collidin; und
aliphatische Sulfone, vorzugsweise Sulfolan.

Die organischen Lösungsmittel können sowohl einzeln als such in Mischung eingesetzt werden.

Besonders bevorzugte Lösungsmittel sind flüssiges $SO_2$ und Methylenchlorid.

Die Menge des eingesetzten inerten Lösungsmittels ist nicht kritisch. Wenn ein Lösungsmittel eingesetzt wird, soll lediglich eine ausreichende Lösung der Reaktanten gewährleistet sein; nach oben ist die Menge des Lösungsmittels von Wirtschaftlichkeitserwägungen begrenzt. Günstige Konzentrationen liegen bei etwa 5 bis 50 Gew.-%, vorzugsweise etwa 15 bis 30 Gew.-% $SO_3$.

In einer bevorzugten Ausführungsform des erfindungsgemäßen Verfahrens wird sowohl für die Acetoacetamid-N-sulfonsäure bzw. deren Salze als auch für das $SO_3$ das gleiche inerte Lösungsmittel, vorzugsweise aus der Gruppe der halogenierten aliphatischen Kohlenwasserstoffe, insbesondere nur Methylenchlorid, verwendet.

Bezüglich des Molverhältnisses von Acetoacetamid-N-sulfonsäure bzw. -sulfonat und $SO_3$ gilt im Prinzip das gleiche was auch bei Stufe b) des Verfahrens der vorerwähnten Patentanmeldung ausgesagt ist. Bevorzugt wird ein bis zu 20-facher $SO_3$-Überschuß, vorzugsweise ein 3- bis 10-facher, insbesondere 4- bis 7-facher molarer Überschuß eingesetzt.

Auch die Reaktionstemperatur für die Ringschlußreaktion liegt in dem in der vorerwähnten Patentanmeldung angegebenen Rahmen von etwa −70 bis +175°C, wobei für die erfindungsgemäße Kurzzeit-Variante Temperaturen zwischen etwa −30 und +100°C bevorzugt sind.

Die Durchführung der erfindungsgemäßen Ringschlußreaktion erfolgt in der Weise, daß die Massenströme aus Acetoacetamid-N-sulfonsäure- oder -sulfonat-Lösung und $SO_3$ oder $SO_3$-Lösung im entsprechenden molaren Verhältnis der Reaktionspartner zusammengeführt und intensiv gemischt werden, die Reaktionswärme abgeführt und nach kurzer Verweilzeit das Reaktionsgemisch ggf. der Hydrolyse zugeführt wird.

Als Vorrichtungen für die Ringschlußreaktion eignen sich im Prinzip alle Vorrichtungen, in denen schnell und unter Wärmeentwicklung ablaufende Reaktionen durchgeführt werden können.

Hohe Ausbeuten werden wegen der kurzen Reaktionszeit und hohen Wärmetönung bevorzugt in speziellen Vorrichtungen erzielt.

Erfindungsgemäß werden daher vorzugsweise als spezielle Vorrichtungen besondere Reaktoren, gegebenenfalls unter Verwendung von Verdampfungskühlung, verwendet, wie z.B. Dünnschichtreaktoren mit mechanischem Rührer, Fallfilmreaktoren, Sprühreaktoren, Rohrreaktoren mit oder ohne Einbauten. Im Dünnschichtreaktor führt man z.B. die beiden Massenströme vorzugsweise voneinander getrennt in den Reaktionsraum ein, indem man sie entweder auf Wischerachse und Mantel leitet oder an verschiedenen Stellen des Mantels einschleust.

Die Wärmeabfuhr kann durch Außenkühlung and/oder durch Verdampfen des Lösungsmittels erfolgen. Da die exotherme Reaktion in Abwesenheit einer Außenkühlung die Temperatur bis zum Siedepunkt des verwendeten Lösungsmittels ansteigen läßt, beträgt die Reaktionstemperatur z.B. im Falle des als Lösungsmittel bevorzugten Methylenchlorids etwa 40°C (= Siedepunkt des Methylenchlorids) bei Atmosphärendruck. Durch Anlegen von Vakumm kann das Verdampfen und damit die Kühlung noch beschleunigt werden; durch Einstellung eines bestimmten Druckes im Reaktor läßt sich die gewünschte Reaktionstemperatur regeln.

Das Reaktionsprodukt der Ringschlußreaktion (hauptsächlich "$SO_3$-Addukt") kann nach — möglichst nicht allzulanger — Zwischenlagerung oder auch unmittelbar nach Beendigung der Ringschlußreaktion hydrolysiert werden; bevorzugt ist die Hydrolyse in unmittelbarem Anschluß in die Ringschlußreaktion.

Die Hydrolyse erfolgt allgemein durch Vermischen des Reaktionsprodukts der Ringschlußstufe mit Wasser. Die Menge des zugesetzten Wassers richtet sich nach dem für die Ringschlußreaktion eingesetzten Überschuß an $SO_3$ und wird zweckmäßig so bemessen, daß nach der Hydrolyse eine wässrige $H_2SO_4$-Lösung einer Konzentration zwischen etwa 10 bis 90 Gew.-%, vorzugsweise von etwa 50 bis 70 Gew.-%, entsteht.

Bei der näheren Untersuchung der Hydrolyse wurde gefunden, daß sie praktisch ebenso schnell wie die Ringschlußreaktion verläuft. Die Hydrolysezeiten liegen also in der gleichen Größenordnung wie die Reaktionszeiten für den Ringschluß. Es ist also möglich, die Hydrolyse — ebenso wie die Ringschlußreaktion — in Zeiten von höchstens etwa 10 Minuten, vorzugsweise von etwa 0,001 bis 60 Sekunden, insbesondere in etwa 0,01 bis 10 Sekunden, durchzuführen.

Aufgrund dieser kurzen Hydrolysezeiten ist es auch nicht unbedingt erforderlich, bei der Hydrolyse möglichst niedrige Temperaturen einzuhalten, so daß eine Kühlung auf tiefem Temperaturniveau hier überflüssig ist. Bei den kurzen Hydrolysezeiten liegen die Reaktionstemperaturen im allgemeinen zwischen etwa −10 und +100°C, vorzugsweise zwischen etwa 0 und 50°C. Die Gefahr einer etwaigen thermischen Zersetzung des bei der Hydrolyse gebildeten 6-Methyl-3,4-dihydro-1,2,3-oxathiazin-4-on-2,2-dioxids besteht hierbei praktisch nicht.

Aufgrund des ähnlichen Verfahrensablaufs von Ringschlußreaktion und Hydrolyse kann die Hydrolyse mit Vorteil auch in Vorrichtungen der gleichen Art wie die Ringsschlußreaktion durchgeführt werden (Dünnschicht-, Fallfilm-, Sprüh-Reaktoren, Strömungsrohre etc.); auch der Einsatz von üblichen Reaktionsgefäßen wie z.B. (Rühr-)Kessel, Kaskade etc. ist jedoch möglich.

Die Isolierung des 6-Methyl-3,4-dihydro-1,2,3-oxathiazin-4-on-2,2-dioxids aus der Produktlösung der Hydrolyse erfolgt nach üblichen Methoden wie sie z.B. in der vorerwähnten Patentanmeldung beschrieben sind, oder es erfolgt gleich die Umsetzung zu den entsprechenden Salzen und deren Isolierung. Die Ausbeuten von Ringschlußreaktion und Hydrolyse liegen in der gleichen Größenordnung wie dies bereits in der vorerwähnten Patentanmeldung für die Stufe b) des dort beschriebenen Verfahrens angegeben ist. Infolge der kurzen Reaktionszeiten für Ringschlußreaktion und Hydrolyse und der geringen Apparatedimensionen der für derartige Kurzzeitreaktionen verwendeten Vorrichtungen sind jedoch die Raum-Zeit-Ausbeuten gegenüber den Raum-Zeit-Ausbeuten, welche nach der in der vorerwähnten Patentanmeldung beschriebenen Verfahrensweise erhalten werden, beträchtlich erhöht.

Es ist möglich, die Hydrolyse in diskontinuierlichem Betrieb durchzuführen; bevorzugt ist jedoch die kontinuierliche Verfahrensweise sowohl für die Hydrolyse als auch für die Ringschlußreaktion.

Einige beispielhafte Ausführungsformen der kontinuierlichen Verfahrensweise sind in den Figuren schematisch dargestellt.

Figur 1 zeigt eine Anordnung aus zwei hintereinander geschalteten Dünnschichtreaktoren 1 und 2. Die Lösungen von Acetoacetamid-N-sulfonsäure bzw. -sulfonat und von $SO_3$ (z.B. in Methylenchlorid) werden über die Leitungen 3 und 4 getrennt in den ersten Reaktor 1 eindosiert. Mit einem mechanischen Rührer 5 werden die Komponenten intensiv durchmischt. Nach der (Ringschluß-)Reaktion läuft das Produkt über die Verbindung 6 aus dem ersten Reaktor 1 in den zweiten 2 — den Hydrolysereaktor ab. In diesem — der in gleicher Weise wie der Reaktor 1 beschaffen ist — wird über die Leitung 7 Wasser ($H_2O$) zudosiert. Das Hydrolyseprodukt läuft über die Leitung 8 ab und wird der Aufarbeitung zugeführt. Die Wärmeabfuhr geschieht durch Kühlung der Reaktorwände 9 mit einem Kälteträger über die Zuleitungen 10 und die Ableitungen 11.

Nach figur 2 werden beide Reaktionen — Ringschlußreaktion und Hydrolyse — in einem einzigen Dünnschichtreaktor 12 durchgeführt, der — ebenso wie die 2 in Figur 1 dargestellten Reaktoren — mit einem Rührer 5 ausgestattet ist. Bei dieser Ausführung entfällt die für den Produkttransport zwischen den Reaktoren gemäß Figur 1 benötigte Zeit. Die Einsatzstoffe Acetoacetamid-N-sulfonsäure bzw. -sulfonat und $SO_3$ (gelöst z.B. in Methylenchlorid) werden im oberen Teil des Reaktors über die Leitungen 3 und 4 zugeführt; nach Abschluß der Ringschlußreaktion wird die bei der Hydrolyse benötigte Wassermenge über die Leitung 13 zugegeben. Die Abfuhr der entstehenden Reaktionswärme geschieht auch in diesem Fall durch Kühlung der Reaktorwand 9 mit einem Kälteträger über die Zuleitung 10 und die Ableitung 11. Über die Leitung 8 wird das Hydrolyseprodukt abgeführt.

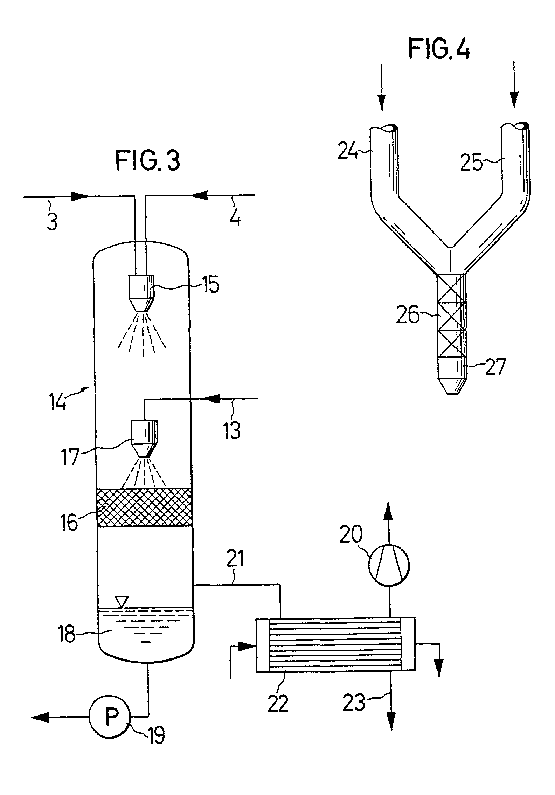

Figur 3 zeigt die Ausführung des erfindungsgemäßen Verfahrens in einem Sprühreaktor 14. Die Lösungen der Ausgangsprodukte Acetoacetamid-N-sulfonsäure bzw. -sulfonat und $SO_3$ werden über die Leitungen 3 und 4 der Misch- und Sprüheinrichtung 15 zugeführt und über diese in den Reaktor eingesprüht. Das Ringschlußprodukt fällt dann auf die Füllkörperschüttung oder Packung 16, auf welche durch die Düse 17 Wasser zum Zwecke der Hydrolyse gesprüht wird; die Zufuhr des Wassers erfolgt über die Leitung 13. Am unteren Ende des Reaktors wird das Hydrolyseprodukt 18 über die Pumpe 19 abgezogen. Über das Vakuumaggregat 20, das mit dem Reaktor 14 durch die Leitung 21 und den Kondensator 22 verbunden ist, wird in dem Reaktor 14 ein Unterdruck zur Verdampfung des Lösemittels erzeugt. Das Lösemittel wird im Kondensator 22 kondensiert und über die Leitung 23 abgezogen.

Figur 4 zeigt eine bevorzugte Ausführungsform der in Figur 3 dargestellten Misch- und Sprüheinrichtung 15. Die Einsatzstoffe werden durch die Leitungen 24 und 25 zugeführt und mit statischen Mischern oder Strahlmischern 26 in Bruchteilen einer Sekunde gemischt und mit der Düse 27 zu Tropfen zerteilt.

Die nachfolgenden Beispiele sollen der weiteren Erläuterung der Erfindung dienen.

Beispiel 1

Kontinuierliche Ringschlußreaktion und Hydrolyse in zwei hintereinander geschalteten Dünnschichtreaktoren.

Herstellung der Ausgangs-Lösungen:

Acetoacetamid-N-sulfonat:

97,1 g (1,0 Mol) Amidosulfonsäure wurden in 1,0 1 Methylenchlorid suspendiert. Unter Rühren wurden 106 g (1,05 Mol) Triethylamin zugefügt, wobei die Amidosulfonsäure als Triethylammoniumsalz in Lösung ging. Nach Zugabe von 6 g (0,1 Mol) Eisessig wurden unter Ruhren bei einet Innentemperatur von 15°C 93,8 g (1,08 Mol) 97 %iges Diketen innerhalb von einer Stunde eingetropft. Nach chromatographischer Analyse die Ausbeute an Acetoacetamid-N-triethylammoniumsulfonat bei 90%. Die so erhaltene Lösung wurde direkte für die weitere Umsetzung eingesetzt.

$SO_3$:

Es wurden 183 g $SO_3$ (2,29 Mol) in 1070 g Methylenchlorid gelöst.

Dünnschichtreaktoren:

Es wurden handelsübliche Labordünnschichtverdampfer der effektiven Länge 22 cm und der Fläche 160 $cm^2$ verwendet. Die Anordnung der Reaktoren entsprach derjenigen gemäß Figur 1.

In den ersten Dünnschichtreaktor wurden bei einer Temperatur des Kälteträgers von −8°C innerhalb von einer Stunde 728 g der — wie vorstehend beschrieben hergestellten — Acetoacetamid-N-sulfonatlösung (mie einem Gehalt von 0,4 Mol des Sulfonats) auf die Achse des Wischers und gleichzeitig die vorstehend beschriebene Lösung von $SO_3$ auf den Reaktormantel gepumpt. Die Drehzahl des Rührers lag bei 1000 Umdrehungen/Minute. Das mit einer Temperatur von 0°C aus dem ersten Reaktor in den Hydrolysereaktor ablaufende Reaktionsprodukt wurde dort bei 0°C hydrolysiert. Zu diesem Zweck wurden innerhalb einer Stunde 200 ml $H_2O$ eingepumpt.

Das aus dem Hydrolysereaktor austretende Produkt wurde aufgearbeitet, indem die organische Phase abgetrennt und die wässrige, schwefelsäurehaltige Phase noch zweimal mit je 1 l Methylenchlorid extrahiert wurde. Die vereinigten organischen Phasen wurden über Natriumsulfat getrocknet und das Methylenchlorid im Vakuum entfernt. Der Rückstand wurde in derse ben Gewichtsmenge Methanol gelöst und anschließend mit 20 %iger KOH/Methanol auf pH 8 bis 10 gebracht. Das ausgefallene Acesulfam K wurde abfiltriert und im Vakuum getrocknet.

Die Ausbeute betrug 60,3 g (0,3 Mol) = 75%, bezogen auf das Acetoacetamid-N-sulfonat.

Die Produktverwei zeit in den Reaktoren war ca. 4,2 sec/Reaktor.

Aus den Volumenströmen der Einsatzlösungen, dem erhaltenen Acesulfam K und den reaktordimensionen errechnet sich eine Raum-Zeit-Ausbeute vom 325 g Acesulfam $K/l\cdot_n$.

Beispiel 2

Durchführung von Ringschlußreaktion und Hydrolyse in ein-und demselben (Dünnschicht-)Reaktor entsprechend Figur 2.

In einen Dünnschichtreaktor der in Beispiel 1 beschriebenen Art und Abmessungen werden bei einer Temperatur des Kühlmediums von −8°C die in Beispiel 1 angegebenen Mengen der Reaktionspartner eingespeist. Gleichzeitig wurde — wie in Figur 2 dargestellt — das Wasser dür die Hydrolyse über einen in der Mitte des Reaktors angebrachten Stutzen eingepumpt.

Nach der Aufarbeitung wie in Beispiel 1 wurde Acesulfam K in einer Ausbeute von 74%, bezogen auf das Acetoacetamid-N-sulfonat, isoliert.

Die Produktverweilzeit betrug im Ringschluß- und im Hydrolyseteil jeweils ca. 2 Sekunden.

Aus den Volumenströmen der Einsatzlösungen, dem erhaltenen Acefulfam K und den Reaktordimensionen errechnet sich eine Raum-Zeit-Ausbeute von 640 g Acesulfam $K/l\cdot_n$.

Beispiel 3

Durchführung von Ringschlußreaktion im Dünnschichtreaktor und der Hydrolyse im Rundkolben.

Für die Ringschlußreaktion wurde ein Dünnschichtreaktor der in Beispiel 1 beschriebenen Art und Abmessungen eingesetzt. Die Hydrolyse wurde in einem darunter angeordneten Rundkolben mit vorgelegtem Wasser durchgeführt.

Es wurde eine 0,41 molare Acetoacetamid-N-sulfonat-Lösung und eine 7,5 (Gew.)%ige Lösung von $SO_3$ in Methylenchlorid verwendet. Beide Einsatzlösungen wurden auf −30°C vorgekühlt. Die Temperatur des Kühlmediums für den Dünnschichtreaktor lag bei −36°C.

Die Hydrolyse wurde bei 0°C vorgenommen.

Die Details und Ergebnisse einiger verschiedener Versuchsläufe sind aus der nachstehenden Tabelle ersichtlich. Die Verweilzeiten im Dünnschichtkreaktor wurden aus gemessenen Werten des im Reaktor enthaltenen Produktvolumens und des Volumenstroms errechnet.

TABELLE

| Versuch | Durchsatz Acetoacetamid-N-Sulfonat-Lösung (mol/min) | Durchsatz $SO_3$-Lösung (mol/min) | Ausbeute an Acesulfam K (%) | Verweilzeit im Dünnschicht-reaktor (sec.) |
|---|---|---|---|---|
| a | 0,1/40 | 0,57/40 | 76 | 5,7 |
| b | 0,1/20 | 0,57/20 | 76 | 2,8 |
| c | 0,1/12,5 | 0,57/12,5 | 75 | 1,8 |
| d | 0,1/10 | 0,57/10 | 75 | 1,4 |
| e | 0,1/4 | 0,57/4 | 70 | 0,7 |
| f | 0,1/2 | 0,57/2 | 70 | 0,3 |

Beispiel 4

Durchführung der Ringschlußreaktion im Dünnschichtreaktor und der Hydrolyse im Rundkolben unter Siedekühlung im Vakuum.

Für die Ringschlußreaktion wurde ein Dünnschichtreaktor der in Beispiel 1 beschriebenen Art und Abmessungen eingesetzt.

Die Hydrolyse wurde in einem unter dem Reaktor angebrachten Rundkolben mit vorgelegtem Wasser durchgeführt. Über das Hydrolysegefäß wurde das Vakuum angelegt.

Es wurden die in Beispiel 1 beschriebenen Einsatzlösungen in den dort angegebenen Mengenverhältnissen eingesetzt. Die Details und Ergebnisse einiger verschiedener Versuchsläufe sind aus der nachstehenden Tabelle ersichtlich.

TABELLE

| Versuch | Druck (mbar) | Ablauf Temp. (°C) | Durchsatz bezogen auf Acetoacetamid-N-sulfonat (Mol/min) | Ausbeute an Acesulfam K (%) | Verweilzeit im Dünnschicht-reaktor (sec.) |
|---|---|---|---|---|---|
| a | 35 | −30 | 0,2/30 | 78 | 5,6 |
| b | 100 | −10 | 0,2/29 | 72 | 5,5 |
| c | 100 | −10 | 0,2/6,25 | 75 | 1,2 |
| d | 180 | 0 | 0,2/30 | 68 | 5,6 |
| e | 180 | 0 | 0,2/9,75 | 70 | 1,9 |
| f | 180 | 0 | 0,2/5,5 | 70 | 1,1 |
| g | 180 | 0 | 0,2/3 | 73 | 0,5 |
| h | 180 | 0 | 0,2/2 | 74 | 0,4 |

Beispiel 5

Durchführung von Ringschlußreaktion und Hydrolyse in zwei in Reihe angeordneten Dünnschichtreaktoren gemäß Figur 1.

Eine 17 (gew.)%ige Acetoacetamid-N-sulfonat- und eine 16 (gew.)%ige $SO_3$-Lösung in Methylenchlorid wurden auf −25°C vorgekühlt. In den ersten Reaktor (Typ Sambay), dessen Wände mit Sole von ebenfalls −25°C gekühlt wurden, wurde die Acetoacetamid-N-sulfonat-Lösung mit einer Geschwindigkeit entsprechend 48 Mol Acetoacetamid-N-sulfonat/Stunde, und die $SO_3$-Lösung mit einer Geschwindkigkeit entsprechend 288 Mol $SO_3$/Stunde eindosiert. Die Wischerumfangsgeschwindigkeit betrug 2,2 m/sec.

Die den ersten Reaktor verlassende Produkt wurde unmittelbar in den zweiten Reaktor — zusammen mit 28 kg $H_2O$ von Raumtemperatur/Stunde — eingeführt. Die Wischerumfangsgeschwindigkeit in diesem Reaktor war die gleiche wie im ersten Reaktor.

Die Aufarbeitung des den zweiten Reaktor verlassenden Produkts geschah durch Abtrennung der organischen Phase von der wässrigen schwefelsauren Phase bei 0 bis 5°C, nochmalige Extraktion der wässrig-schwefelsauren Phase mit Methylenchlorid.

Trocknung der vereinigten organischen Phase über $Na_2SO_4$ und Entfernung des Methylenchlorids im Vakuum, Lösen des Rückstandes in derselben Gewichtsmenge Methanol und anschließend Versetzen mit 20% iger KOH/Methanol bis auf pH 8—10. Das ausgefallene Acesulfam K wurde abfiltriert und im Vakuum getrocknet. Die Reaktionsausbeute betrug 73% d.Th.

Die Produktverweilzeit bei der Ringschlußreaktion im ersten Reaktor betrug bei Schichtdicken von 1 mm Stärke 5 Sekunden.

Beispiel 6

Durchführung der Ringschlußreaktion im Sprühreaktor und der Hydrolyse im Rührkolben.

In einem Glasrohr (Innendurchmesser 10 mm) mit eingebauten statischen Mischern wurden gleiche Volumenströme einer 0,7 molaren (ca. 15 %igen) Lösung von Acetoacetamid-N-sulfonat sowie einer 4,2 molaren (ca. 23 %igen) $SO_3$-Lösung im Methylenchlorid kontinuierlich gemischt und in einen Rührkolben gesprüht. Im Rührkolben wurde durch kontinuierliche Wasserzufuhr hydrolysiert. Die Abfuhr der Reaktionswärme geschah bei der Ringschlußreaktion und bei der Hydrolyse durch Verdampfungskühlung bei 40°C. Die Produktverweilzeit betrug bei der Ringschlußreaktion ca. 0,05 Sekunden. Nach Aufarbeitung gemäß Beispiel 1 wurde eine Ausbeute an Acesulfam K von 67% erhalten.

Die errechnete Raum-Zeit-Ausbeute betrug 3000 kg Acesulfam K/h·l.

**Patentansprüche**

1. Verfahren zur Herstellung von 6-Methyl-3,4-dihydro-1,2,3-oxothiazin-4-on-2,2-dioxid durch Ringschluß eines Acetoacetamid-Derivats, dadurch gekennzeichnet, daß man als Acetoacetamid-Derivat Acetoacetamid-N-sulfonsäure oder deren Salze — gelöst in einem inerten Lösungsmittel — verwendet, daß man den Ringschluß durch die Einwirkung einer mehr als äquimolaren Menge $SO_3$ — gegebenenfalls in gleicher Weise gelöst in einem inerten Lösungsmittel — in einer Reaktorverweilzeit von höchstens 10 Minuten durchführt und daß man das als $SO_3$-Addukt anfallende 6-Methyl-3,4-dihydro-1,2,3-oxathiazin-4-on-2,2-dioxid hydrolysiert.

2. Verfahren nach Anspruch 1, dadurch gekennzeichnet, daß man den Ringschluß in einer Reaktorverweilzeit von 0,001 bis 60 Sekunden insbesondere 0,1 bis 10 Sekunden, durchführt.

3. Verfahren nach Anspruch 1 oder 2, dadurch gekennzeichnet, daß man Lösungen der Acetoacetamid-N-sulfonsäure oder von deren Salzen und von $SO_3$ in dem gleichen inerten Lösungsmittel, vorzugsweise einem aliphatischen Chlorkohlenwasserstoff, insbesondere Methylenchlorid, verwendet.

4. Verfahren nach einem oder mehreren der Ansprüche 1 bis 3, dadurch gekennzeichnet, daß man den Ringschluß bei Temperaturen zwischen −30 und +100°C durchführt.

5. Verfahren nach einem oder mehreren der Ansprüche 1 bis 4, dadurch gekennzeichnet, daß man die Ringschlußreaktion in einem Dünnschicht-, Fallfilm- oder Sprühreaktor oder in einem Strömungsrohr mit oder ohne Einbauten durchführt.

6. Verfahren nach einem oder mehreren der Ansprüche 1 bis 5, dadurch gekennzeichnet, daß man die bei der Ringschlußreaktion freiwerdende Wärme durch die Verdampfung des Lösungsmittels, gegebenenfalls im Vakuum, abführt.

7. Verfahren nach einem oder mehreren der Ansprüche 1 bis 6, dadurch gekennzeichnet, daß man die Hydrolyse des $SO_3$-Addukts des 6-Methyl-3,4-dihydro-1,2,3-oxathiazin-4-on-2,2-dioxids im Anschluß an die Ringschlußreaktion durchführt.

8. Verfahren nach einem oder mehreren der Ansprüche 1 bis 7, dadurch gekennzeichnet, daß man die Hydrolyse in Reaktorverweilzeiten der gleichen Größenordnung wie den Ringschluß durchführt.

9. Verfahren nach einem oder mehreren der Ansprüche 1 bis 8, dadurch gekennzeichnet, daß man die Hydrolyse bei Temperaturen zwischen −10 und +100°C, vorzugsweise zwischen 0 und +50°C, durchführt.

10. Verfahren nach einem oder mehreren der Ansprüche 1 bis 9, dadurch gekennzeichnet, daß man die Hydrolyse in Vorrichtungen der gleichen Art wie die Ringschlußreaktion durchführt.

11. Verfahren nach einem oder mehreren der Ansprüche 1 bis 10, dadurch gekennzeichnet, daß man Ringschluß und Hydrolyse kontinuierlich durchführt.

12. Verfahren nach einem oder mehreren der Ansprüche 1 bis 11, dadurch gekennzeichnet, daß man fürden Ringschluß einen bis zu 20fachen molaren $SO_3$-Überschuß, vorzugsweise einen 3- bis 10fachen, insbesondere 4- bis 7fachen molaren $SO_3$-Überschuß verwendet.

**Revendications**

1. Procédé pour préparer le méthyl-6 dihydro-3,4 oxathiazine-1,2,3 one-4 dioxyde-2,2 par cyclisation d'un dérivé de l'acétoacétamide, procédé caractérisé en ce qu'on utilise, comme dérivé de

l'acéatoacétamide, l'acide acétoacétamide-N-sulfonique ou l'un de ses sels, dissous dans un solvant inerte, en ce qu'on effectue la cyclisation en faisant agir une quantité de $SO_3$ supérieure à la quantité équimolaire — éventuellement dissous de la même façon dans un solvant inerte -avec un temps de séjour dans la réacteur d'au plus 10 minutes, et en ce qu'on hydrolyse le méthyl-6 dihydro-3,4 oxathiazine-1,2,3 one-4 dioxyde-2,2 obtenu sous la forme de son produit d'addition avec $SO_3$ ("$SO_3$-adduit").

2. Procédé selon la revendication 1, caractérisé en ce qu'on effectue la cyclisation avec un temps de séjour dans la réacteur de 0,001 à 60 secondes, plus particulièrement de 0,01 à 10 secondes.

3. Procédé selon l'une des revendications 1 et 2, caractérisé en ce qu'on utilise des solutions d'acide acétoacétamide-N-sulfonique, ou d'un de ses sels, et de $SO_3$ dans le même solvant inerte, de préférence dans un hydrocarbure chloré aliphatique, plus particulièrement le chlorure de méthylène.

4. Procédé selon l'une quelconque des revendications 1 à 3 caractérisé en ce qu'on effectue la cyclisation à des températures comprises entre −30 et +100°C.

5. Procédé selon l'une quelconque des revendications 1 à 4, caractérisé en ce qu'on effectue la réaction de cyclisation dans un réacteur à couche mince, à pellicule tombante ou à pulverisation ou dans un tube d'écoulement avec ou sans chicanes.

6. Procédé selon l'une quelconque des revendications 1 à 5, caractérisé en ce que la chaleur libérée lors de la réaction de cyclisation est éliminée par évaporation du solvant, éventuellement sous pression réduite.

7. Procédé selon l'une quelconque des revendications 1 à 6, caractérisé en ce que l'hydrolyse du "$SO_3$-adduit" du méthyl-6 dihydro-3,4, oxathiazine-1,2,3 one-4 dioxyde-1,1 est effectuée à la suite de la réaction de cyclisation.

8. Procédé selon l'une quelconque des revendications 1 à 7, caractérisé en ce que l'hydrolyse est effectuée avec un temps de séjour dans le réacteur qui est du même ordre de grandeur que pour la cyclisation.

9. Procédé selon l'une quelconque des revendications 1 à 8, caractérisé en ce que l'hydrolyse est effectuée à des températures comprises entre −10 et +100°C, de préférence entre 0 et +50°C.

10. Procédé selon l'une quelconque des revendications 1 à 9, caractérisé en ce que l'hydrolyse est effectuée dans des appareils du même type que ceux qui servent pour la réaction de cyclisation.

11. Procédé selon l'une quelconque des revendications 1 à 10, caractérisé en ce qu'on effectue la cyclisation et l'hydrolyse en continu.

12. Procédé selon l'une quelconque des revendications 1 à 11, caractérisé en ce qu'on utilise, pour la cyclisation, un excès de $SO_3$ pouvant aller jusqu'à 20 fois la quantité molaire, de préférence de 3 à 10 fois, plus particulièrement de 4 à 7 fois la quantité molaire.

**Claims**

1. A process for the preparation of 6-methyl-3,4-dihydro-1,2,3-oxathiazin-4-one 2,2-dioxide by ring closure of an acetoacetamide derivative, characterized by using, as acetoacetamide derivative, acetoacetamide-N-sulfonic acid or its salts — dissolved in an inert solvent — and

by the ring closure being carried out by the action of more than an equimolar amount of $SO_3$ — where appropriate dissolved in an inert solvent in the same way — in a reactor residence time not exceeding 10 minutes, and

by the 6-methyl-3,4-dihydro-1,2,3-oxathiazin-4-one 2,2-dioxide which results at the $SO_3$ adduct being hydrolyzed.

2. The process as claimed in claim 1, wherein the ring closure is carried out in a reactor residence time of 0.001 to 60 seconds, in particular 0.01 to 10 seconds.

3. The process as claimed in claim 1 or 2, wherein solutions of acetoacetamide-N-sulfonic acid or of its salts and of $SO_3$ in the same inert solvent, preferably a chlorinated aliphatic hydrocarbon, in particular methylene chloride, are used.

4. The process as claimed in one or more of claims 1 to 3, wherein in the ring closure is carried out at temperatures between −30 and +100°C.

5. The process as claimed in one or more of claims 1 to 4, wherein the ring closure reaction is carried out in a thin-film, falling film or spray reactor or in a flow tube with or without fittings inside.

6. The process as claimed in one or more of claims 1 to 5, wherein the heat liberated in the ring closure reaction is dissipated by evaporation of the solvent, where appropriate in vacuo.

7. The process as claimed in one or more of claims 1 to 6, wherein the hydrolysis of the $SO_3$ adduct of 6-methyl-3,4-dihydro-1,2,3-oxathiazin-4-one 2,2-dioxide is carried out following the ring closure reaction.

8. The process as claimed in one or more of claims 1 to 7, wherein the hydrolysis is carried out in reactor residence times of the same order of magnitude as the ring closure.

9. The process as claimed in one or more of claims 1 to 8, wherein the hydrolysis is carried out at temperatures between −10 and +100°C, preferably between 0 and +50°C.

10. The process as claimed in one or more of claims 1 to 9, wherein the hydrolysis is carried out in apparatus of the same type as the ring closure reaction.

11. The process as claimed in one or more of claims 1 to 10, wherein the ring closure and hydrolysis are carried out continuously.

12. The process as claimed in one or more of claims 1 to 11, wherein an up to 20-fold molar excess of $SO_3$, preferably a 3- to 10-fold, in particular 4- to 7-fold, molar of $SO_3$ is used.

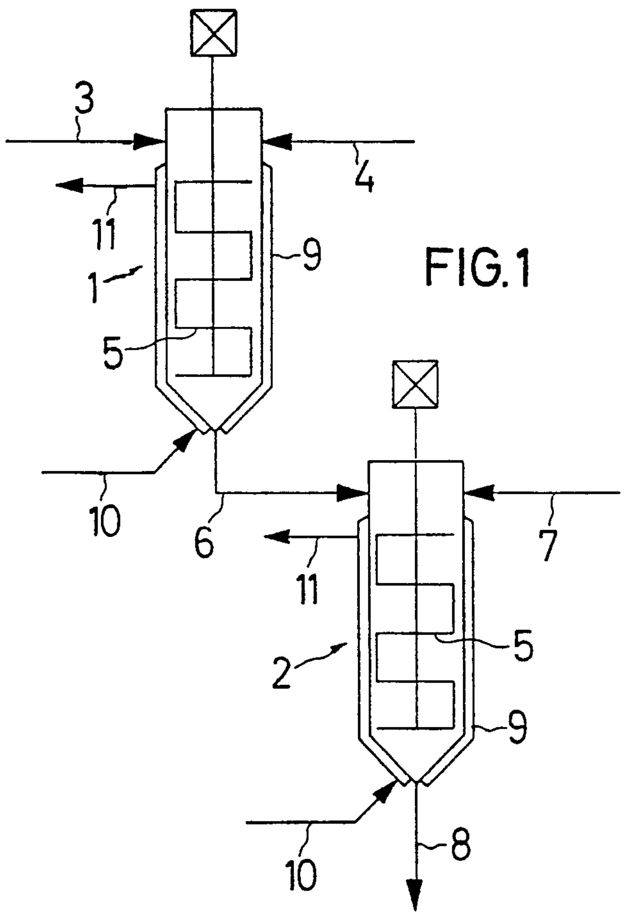


FIG.1

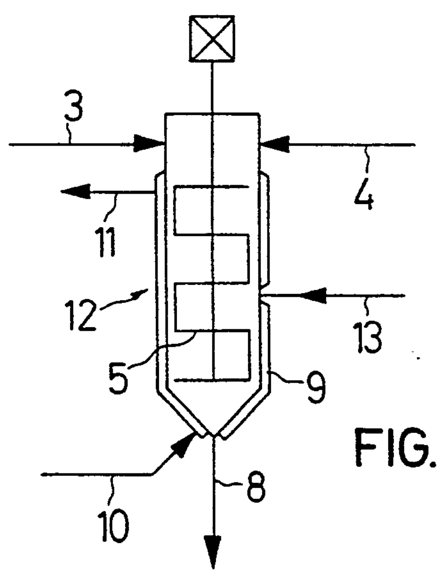


FIG.2

FIG.4

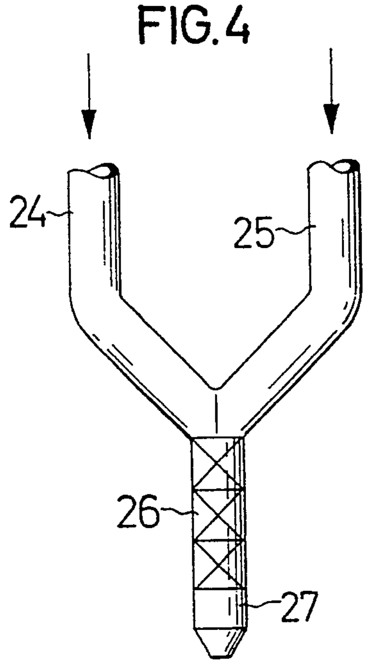


FIG. 3